(19) Europäisches Patentamt
European Patent Office
Office européen des brevets

(11) **EP 3 721 866 A1**

(12) **EUROPEAN PATENT APPLICATION**
published in accordance with Art. 153(4) EPC

(43) Date of publication:
**14.10.2020 Bulletin 2020/42**

(51) Int Cl.:
*A61K 8/81* *(2006.01)*          *A61K 8/02* *(2006.01)*
*A61Q 5/00* *(2006.01)*          *A61Q 19/00* *(2006.01)*

(21) Application number: **17934057.5**

(22) Date of filing: **08.12.2017**

(86) International application number:
**PCT/JP2017/044249**

(87) International publication number:
**WO 2019/111415 (13.06.2019 Gazette 2019/24)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicant: **Wamiles Cosmetics Inc.
Yokohama-shi, Kanagawa 233-0004 (JP)**

(72) Inventors:
• **KAWAGUCHI, Haruma**
  **Yokohama-shi**
  **Kanagawa 233-0004 (JP)**
• **KATO, Nobuhiro**
  **Yokohama-shi**
  **Kanagawa 233-0004 (JP)**
• **HIRATA, Naoyuki**
  **Yokohama-shi**
  **Kanagawa 233-0004 (JP)**

(74) Representative: **Cabinet Beau de Loménie
158, rue de l'Université
75340 Paris Cedex 07 (FR)**

(54) **CATIONIZED VESICLES AND COMPOSITION THEREOF**

(57)    Provided is a vesicle composition that has adsorption properties to the skin and hair, that can encapsulate various active ingredients, and that is suitable for addition to cosmetics.

Cationized vesicles cationized with a cationic polymer that is a polymer of 2-methacryloyloxyethyl phosphorylcholine and a specific cationic monomer; cationized vesicles encapsulating an active ingredient in which the active ingredient is for cosmetics and is held by the cationized vesicles; and a cosmetic containing either of these vesicles are provided.

EP 3 721 866 A1

**Description**

TECHNICAL FIELD

The present invention relates to cationized vesicles cationized with a novel cationic substance and a production method therefor. In particular, the present invention relates to the use of the cationized vesicles as cosmetics.

BACKGROUND ART

A vesicle is a structure enclosed by a bilayer and is formed through self-assembly of amphiphilic molecules. A vesicle formed from a phospholipid bilayer similar to a cell membrane is specially called a liposome. Such vesicles (liposomes) can encapsulate various substances. Liposomes are thus usable for delivery of substances encapsulated therein into cells and are under research and development as carriers in pharmaceutical delivery techniques (DDS: drug delivery systems).

Cosmetics that adopt techniques similar to DDS using liposomes are advertised as nanocosmetics, nanocapsules, and the like. Japanese Patent No. 5623076 (Patent Literature (PTL) 1) describes a vesicle composition containing a lysophospholipid and an external preparation for the skin comprising the vesicle dispersion composition. Japanese Unexamined Patent Application Publication No. 2012-206948 (PTL 2) describes a liposome composition that is stable even in an acidic region and a cosmetic and an external preparation for the skin containing the composition. Japanese Unexamined Patent Application Publication No. 2007-176820 and No. 2007-176821 (PTL 3 and 4) describe preparation of a cosmetic by using a powder phospholipid composition that can form stable liposomes through dispersing in an aqueous medium.

Compositions used through topical application, such as cosmetics, are well designed to enhance penetration of active ingredients into cells.

Cationization herein means positively charging a substance. Since negatively charged, the skin readily adsorbs a positively charged substance.

Japanese Patent No. 4950419 and No. 5220546 (PTL 5 and 6) describe liposomes whose components are designed to stimulate intracellular penetration of a substance in cells of the skin or hair for the purpose of cosmetic uses.

Japanese Unexamined Patent Application Publication No. 2006-35036 (PTL 7) describes oily chemical-containing particles, where the oily chemical is contained in a dispersed state in a water-soluble solid matrix as oil droplets and an emulsifying substance that becomes cationic in water is further contained.

Japanese Unexamined Patent Application Publication No. 2016-108285 (PTL 8) describes cationized vesicles encapsulating minerals as active ingredients for cosmetics. Here, a substance for cationization is preferably cationic hyaluronic acid excellent in adsorption properties to the skin and hair.

CITATION LIST

PATENT LITERATURE

PTL 1: Japanese Patent No. 5623076 "A vesicle composition and an external preparation for the skin"
PTL 2: Japanese Unexamined Patent Application Publication No. 2012-206948 "A liposome composition, a cosmetic and an external preparation for the skin using the same, and a production method therefor"
PTL 3: Japanese Unexamined Patent Application Publication No. 2007-176820 "A powder phospholipid composition"
PTL 4: Japanese Unexamined Patent Application Publication No. 2007-176821 "A powder phospholipid composition"
PTL 5: Japanese Patent No. 4950419 "Liposomes comprising a cationic polymer that stimulates intracellular penetration"
PTL 6: Japanese Patent No. 5220546 "Hydrated lamellar phases or liposomes comprising a fatty monoamine or a cationic polymer that stimulates intracellular penetration, and a cosmetic composition or a pharmaceutical composition comprising the same"
PTL 7 : Japanese Unexamined Patent Application Publication No. 2006-35036 "Oily chemical-containing particles"
PTL 8 : Japanese Unexamined Patent Application Publication No. 2016-108285 "Cationized vesicles and a composition thereof"

SUMMARY OF INVENTION

TECHNICAL PROBLEM

Cationized vesicles are readily adsorbed to the skin and, when used after encapsulating an active ingredient for cosmetics, can efficiently deliver the active ingredient to the skin. For this reason, cationized vesicles are an excellent composition as a cosmetic material. An object of the present invention is to provide cationized vesicles that are highly stable and that can encapsulate not only minerals, but also other various active ingredients for cosmetics.

SOLUTION TO PROBLEM

As a result of extended studies on the composition of a cationic polymer for cationizing vesicles, the present inventors successfully enhanced the stability of cationized vesicles, diversified substances to be encapsulated, and increased the concentration of substances to be encapsulated by using a novel cationic polymer that is different in the composition from conventional cationic polymers, thereby completing the present invention.
Specifically, the present invention relates to cationized vesicles that are cationized with a novel cationic polymer and that are used for cosmetics through addition thereto. The present invention also relates to a method of cationizing vesicles using the novel cationic polymer. Moreover, the present invention relates to a production method for the cationized vesicles by using the novel cationic polymer. Further, the present invention relates to the novel cationic polymer.
It is preferable that the cationic polymer contains 2-methacryloyloxyethyl phosphorylcholine and a cationic monomer, and a proportion of the cationic monomer in the cationic polymer is 50% or more.
The cationic monomer is preferably (2-hydroxy-3-methacryloyloxypropyl)trimethylammonium chloride; [2-(methacryloyloxy)ethyl]trimethylammonium chloride; or N,N-dimethylamino ethylacrylate, benzyl chloride quarternary.
The cationized vesicles may be either unilamellar vesicles or multilamellar vesicles.
The present invention also relates to cationized vesicles encapsulating an active ingredient, where the active ingredient is for cosmetics and is held by the above-mentioned cationized vesicles.
The active ingredient is preferably a negatively charged substance, and examples include minerals, amino acids, anionic surfactants, and vitamins (vitamin C and derivatives thereof, for example).
The present invention also relates to the cationized vesicles, where a zeta potential is +20 mV or more.
The present invention also relates to the cationized vesicles encapsulating an active ingredient, where a zeta potential is +20 mV or more.
The present invention also relates the cationized vesicles, where the vesicles are freeze-dried.
The present inventions also relates to the cationized vesicles encapsulating an active ingredient, where the vesicles are freeze-dried.
Moreover, the present invention relates a cosmetic comprising the above-mentioned cationized vesicles or cationized vesicles encapsulating an active ingredient; or the above-mentioned freeze-dried cationized vesicles or cationized vesicles encapsulating an active ingredient.
The cosmetic may further comprise a substance derived from a natural product.
The present invention also relates to a cosmetic that has adsorption properties to the skin and hair and is applied topically, where the cosmetic is characterized by comprising the above-mentioned cationized vesicles or cationized vesicles encapsulating an active ingredient; or the above-mentioned freeze-dried cationized vesicles or cationized vesicles encapsulating an active ingredient.
Further, the present invention relates a method for a beauty treatment comprising applying to the skin or hair a cosmetic comprising the above-mentioned cationized vesicles or cationized vesicles encapsulating an active ingredient; or the above-mentioned freeze-dried cationized vesicles or cationized vesicles encapsulating an active ingredient.
The method for a beauty treatment may be performed in a salon or at home.

ADVANTAGEOUS EFFECTS OF INVENTION

The cationized vesicles of the present invention exhibit excellent skin permeation/skin penetration and low skin irritation and are thus suitable for addition to functional cosmetics. Moreover, in the cationized vesicles of the present invention, the stability is enhanced, the concentration of substances to be encapsulated (active ingredients, such as minerals) is increased, and substances to be encapsulated are diversified compared with conventionally known vesicles.
According to the present invention, it is possible to provide cosmetics excellent in adsorption, permeation, and sustained efficacy.

DESCRIPTION OF EMBODIMENTS

1. Vesicles

A vesicle is formed as a spherical structure in which a bilayer membrane is closed through spontaneous assembly of amphiphilic molecules in water with the hydrophobic tails inside and the hydrophilic heads outside.

A single-lamellar vesicle (SLV) is also referred to as "unilamellar vesicle" and is a spherical vesicle formed from one lipid bilayer membrane.

A vesicle having a multilayered structure of bilayers is referred to as "multilamellar vesicle (MLV)." The MLV is formed from a plurality of concentrically arranged lipid bilayer membranes.

Vesicles can be formed from various amphiphilic molecules, and those formed from phospholipids are specially called "liposomes." The "vesicle" is a generic term for those formed from phospholipids or surfactants. Liposomes are like capsules having the same membranes as cells in the skin and are excellent in biocompatibility.

Phospholipid membranes are formed from phospholipids or glycerophospholipids derived from egg yolk or soybean; or cyclic phosphatidic acids and are negatively charged originally. Such phospholipids may be synthesized or may be commercial products.

Further, for the purpose of adjusting the hardness and/or penetration of a phospholipid bilayer, it is also possible to combine cholesterol with phospholipids.

Vesicles can be prepared by a conventional production method commonly known as a production method for liposomes.

Examples of publicly known techniques as production methods for liposomes include a method by Bangham et al. (J. Mol. Biol., 13, 238, (1965)) and modified methods thereof; sonication (Biochem. Biophys. Res. Commun., 94, 1367 (1980)); ethanol injection (J. Cell. Biol., 66, 621 (1975)); a French press method (FEBS Lett., 99, 210 (1979)); ether injection (N. Y. Acad. Sci., 308, 250 (1978)); a cholic acid (detergent) method (Biochim. Biophys. Acta, 455, 322 (1976)); calcium fusion (Biochim. Biophys. Acta, 394, 483 (1978)); a freezing and thawing method (Arch. Biochem. Biophys., 212, 186 (1981)); reverse-phase evaporation (Proc. N. A. S. USA, 75, 4194 (1978)); a method using a glass filter (Third US-Japan Symposium on Drug Delivery System (1995)); and a method using a commercial kit, such as Coatsome®.

2. Cationization

Cationization means positively charging a substance. Cationized vesicles are positively charged and are thus expected to be more readily adsorbed to the skin or more readily permeate the skin than non-cationized vesicles. In the present invention, vesicles are desirably cationized such that these effects can be obtained. Specifically, cationized vesicles have a zeta potential of +20 mV or more further preferably +30 mV or more. The zeta potential is desirably maintained in a stable manner even when the cationized vesicles are added with an excipient and freeze-dried for storage, followed by re-dissolution in an aqueous solvent. Without particular distinction, both MLV and SLV are herein referred to as "cationized vesicles" of the present invention. Moreover, cationized MLV and cationized SLV are sometimes referred to as "CMLV" and "CSLV," respectively.

A cationic molecule is used for cationizing vesicles. As such cationic molecules, cationic polymers are commonly used. Cationized vesicles are obtained by adding a cationic molecule (cationic polymer) during preparation of vesicles. A cationic polymer is a polymeric substance cationized by introducing cationic groups. Such a cationic polymer itself needs to be biocompatible and, in addition, preferably has moisturizing effects on the skin and/or hair.

In the present invention, a cationic polymer formed from 2-methacryloyloxyethyl phosphorylcholine (MPC) and a specific cationic monomer is used. MPC, which has the same structure as a cell membrane as well as a polymerizable structure, is an extremely highly biocompatible innovative material.

Examples of the cationic monomer include:

Blemmer® QA: (2-hydroxy-3-methacryloyloxypropyl)trimethylammonium chloride;
MOETAC: [2-(methacryloyloxy)ethyl]trimethylammonium chloride; and
DMAEA®-BQ: N,N-dimethylamino ethylacrylate, benzyl chloride quarternary, or [(2-acryloyloxy)ethyl]benzyldimethylammonium chloride.

An embodiment of the cationic polymer used for cationizing vesicles in the present invention is MPC-Blemmer QA copolymer. This is a synthetic polymer composed of MPC and Blemmer® QA, and the ingredient name (labeling name) is "Polyquaternium-64." Typical commercially available Polyquaternium-64 consists of 30% of MPC and 70% of the cationic monomer.

Moreover, in the present invention, cationic polymers of the novel composition produced in the Examples section are also used suitably. An embodiment of such cationic polymers is a cationic polymer composed of MPC and Blemmer® QA. Another embodiment is a cationic polymer composed of MPC and MOETAC. Still another embodiment is a cationic polymer composed of MPC and DMAEA®-BQ. It is possible to variously change the ratio of MPC to a cationic monomer in a cationic polymer as well as the molecular weight of the cationic polymer. In the present invention, the proportion of a cationic monomer in a cationic polymer is preferably 50% or more and more preferably 55% or more. The present inventors have confirmed that cationized vesicles can be produced even when the proportion of a cationic monomer is increased up to 82%. Moreover, the molecular weight of a cationic polymer is preferably 600,000 or more. The present inventors have confirmed that cationized vesicles can be formed even when the molecular weight is 2,000,000.

3. Use of Cationized Vesicles in Cosmetics

When the cationized vesicles encapsulating an active ingredient for cosmetics are used for cosmetics, such as lotion, essence, milky lotion, cream, and shampoo, through addition thereto, the active ingredient can be efficiently delivered to the skin.

SLV is transparent and is thus suitable for lotion, essence, and the like, whereas MLV is milky white in color and is thus suitable for milky lotion, cream, and the like.

Substances to be encapsulated in the cationized vesicles are appropriately selected depending on uses as cosmetics.

In general, it is difficult to encapsulate negatively charged substances in vesicles or cationized vesicles. However, the cationized vesicles of the present invention can encapsulate negatively charged substances as cosmetic ingredients. Examples of such cosmetic ingredients to be encapsulated include ingredients bearing a negative ion, such as vitamin C and derivatives thereof, anionic surfactants, amino acids, and minerals. Cationized vesicles holding an active ingredient for cosmetics are herein referred to as "cationized vesicles encapsulating an active ingredient."

Examples of the minerals include a concentrated deep ocean water composition described in Japanese Unexamined Patent Application Publication No. 2007-217356, deep ocean water described in Japanese Unexamined Patent Application Publication No. 2000-290159, and an extract of hot-spring sinter described in Japanese Patent No. 3967357. Cationized vesicles encapsulating a concentrated deep ocean water composition, deep ocean water, dried seawater thereof, or an extract of hot-spring sinter are herein referred to as "cationized vesicles encapsulating minerals."

For example, deep ocean water concentrate is NF water concentrate obtained by a production method including steps (1) and (2) below:

(1) step of treating deep ocean water with a nanofiltration membrane that can remove 90% or more sulfate ions to yield NF membrane-permeated water with Brix of 2.8 to 3.6, Mg ion concentration of 300 to 900 mg/L, Ca ion concentration of 200 to 600 mg/L, and $SO_4$ ion concentration of 0 to 300 mg/L; and
(2) step of concentrating the NF membrane-permeated water obtained in step (1) to yield NF water concentrate with Brix of 30.0 to 55.0, Mg ion concentration of 10,000 to 100,000 mg/L, Ca ion concentration of 4,000 to 40,000 mg/L, $SO_4$ ion concentration of 0 to 1,000 mg/L, and Ca ion concentration: Mg ion concentration of 1:0.25 to 1:4.

It is preferable that the above-mentioned concentration of NF membrane-permeated water is 14- to 50-fold concentration and the resulting NF water concentrate is in a state of an aqueous solution without precipitate. Moreover, it is preferable, for example, that the pore size of the nanofiltration membrane is 0.1 to 1 nm and the pressure applied to the membrane is 0.3 to 2.0 MPa.

Further, the extract of hot-spring sinter is a composition containing an ozone oxidation product of hot-spring sinter obtained, for example, by a production method including steps (1) to (3) below:

(1) crystallizing a hot-spring gas on blue clay at a constant temperature and a constant humidity to yield hot-spring sinter;
(2) dissolving the hot-spring sinter in an aqueous solvent; and
(3) subjecting the dissolution solution to ozone oxidation to obtain an ozone oxidation product.

The above-mentioned extract of hot-spring sinter preferably contains (1) 25 to 40,000 mg/L of sulfate ions, (2) 5.0 to 8,000 mg/L of ferric ions, and (3) aluminum ions, where a ratio of ferrous ions to ferric ions is 4,900:2,000 or less. Here, it is also preferable that ferrous ions are 0.5 or less relative to 1 for ferric ions.

Herein, a concentrated deep ocean water composition, deep ocean water, dried seawater, and an extract of hot-spring sinter are simply referred to as "minerals" in some cases.

In the cationized vesicles of the present invention, the concentration of minerals to be encapsulated is increased compared with conventional vesicles. Such minerals can be encapsulated at a concentration of about 0.0001% to about 5%, for example, about 0.1% to about 5%, about 2% to about 5%, or about 3% to about 5% in wt% based on the total weight of vesicles.

Moreover, the cationized vesicles of the present invention can encapsulate various substances. Exemplary cosmetic ingredients to be encapsulated include vitamin C derivatives (sodium ascorbyl phosphate, for example), in addition to the above-described minerals. A vitamin C derivative can be encapsulated at a concentration of about 0.1% to about 5% in wt% based on the total weight of vesicles. The cationized vesicles encapsulating an active ingredient can be produced by a method including stirring and mixing active ingredients to be encapsulated (minerals, amino acids, anionic surfactants, and vitamin C and derivatives thereof, for example), a cationic polymer, a phospholipid, a surfactant, and optionally other cosmetic ingredients, with an aqueous solvent (purified water or seawater) at a temperature of about 80°C (75°C to 85°C, for example), followed by gradual cooling to a temperature ranging from room temperature to about 35°C. In addition, it is also possible to produce the cationized vesicles by appropriately adopting the above-described techniques publicly known as production methods for liposomes. The cooled composition may be further emulsified at a high pressure (220 MPa × 5 passes, for example) and filtered (through 1.0 μm filter, for example).

Examples of other cosmetic ingredients include moisturizing ingredients; crude drug extracts; enzymes, such as tyrosinase, superoxide dismutase, and lipase; vitamins and derivatives thereof, such as retinol, ascorbic acid, tocopherols, pyridoxal, and riboflavin; organic pigments, such as β-carotene and chlorophyll; moisture ingredients, such as glycerol, sorbitol, urea, lactic acid, propylene glycol, polyethylene glycol and copolymers thereof, and glucose derivatives; emollient ingredients, such as paraffin, stearyl alcohol, cetyl alcohol, squalane, silicone oil, and steareth; treatment ingredients; anti-dandruff ingredients; hair growth ingredients; UV absorbers; antioxidants; perfume; and preservatives. Two or more of these ingredients may be used in combination as necessary.

The cosmetics of the present invention can be produced by adding one or more kinds of cationized vesicles encapsulating an active ingredient to common formulations of lotion, essence, milky lotion, cream, shampoo, and the like. Such cosmetics are not particularly limited in their forms but are preferably used through application to the skin or hair. Herein, the use by topical application also includes washing after application to the skin or hair. Exemplary forms include skincare cosmetics, such as facial wash, cleanser, lotion, essence, facial mask, massage cream, milky lotion, moisture cream, and lip balm; makeup cosmetics, such as foundation, face powder, lipstick, cheek rouge, and eyeshadow; bodycare cosmetics, such as soap, liquid soap, and bath additive; and haircare cosmetics, such as shampoo, conditioner, hair treatment, hair liquid, hair tonic, and scalp treatment. Unless desirable effects are lost, the above-mentioned cosmetics may appropriately contain various ingredients commonly used in cosmetics (oil and fat, wax, hydrocarbons, fatty acids, alcohols, esters, amino acids, vitamins, surfactants, pH adjusters, preservatives, perfume, moisturizers, powder, UV absorbers, thickeners, pigments, antioxidants, whitening agents, anti-inflammatory agents, line smoothers, skin roughness improvers, anti-acne agents, alkalis, chelating agents, and sequestrants, for example).

As described in the Examples section hereinafter, the cosmetics of the present invention may contain substances derived from natural products. Examples of such substances include the following: a plum extract, such as a plum root extract and steam distilled water of plum fruit, described in Japanese Patent No. 4795475; fermented plum juice described in Japanese Patent No. 2526362; plant-derived steam distilled water, for example, steam distilled water of one or two or more plants selected from passion fruit, *Citrus depressa,* banana, atemoya, plum, *Citrus tankan,* lemon-lime (hybrid of lemon and lime), and ginger described in Japanese Unexamined Patent Application Publication No. 2012-116761; an *Angelica acutiloba* root extract described in Japanese Patent No. 3544505; and a seawater-derived yeast culture described in Japanese Unexamined Patent Application Publication No. 2009-029788.

EXAMPLES

Hereinafter, the present invention will be described specifically by means of Examples. However, these Examples are for illustrating the present invention and by no means limit the scope of the present invention.

[Example 1: Preparation of Cationic Polymers]

To a four-neck flask, 16.6 g of MPC and 33.4 g of Blemmer® QA dissolved in 200 g of water were fed and, after nitrogen blowing for 30 minutes, polymerized at 75°C for 10 hours by adding a polymerization initiator (VA-061 from Wako Pure Chemical Industries, Ltd., 0.55 g). The polymerization solution was added dropwise to 5 L of diethyl ether while stirring, and the resulting precipitate was obtained through filtration and vacuum-dried at room temperature for 48 hours to yield 30.1 g of cationic polymer powder. The obtained cationic polymer (Polyquaternium-64) had a molecular weight of 700,000 and consisted of 45% of MPC and 55% of Blemmer® QA (MPC/cationic monomer = 45/55). Further, cationic polymers with different molecular weights and MPC to Blemmer QA ratios as well as cationic polymers composed of MPC and a different cationic monomer of MOETAC or DMAEA-BQ were obtained in a similar manner.

[Example 2: Preparation and Stability Evaluation of Cationized Vesicles Encapsulating Minerals]

Cationized vesicles encapsulating minerals were prepared from the following substances:

hydrogenated sodium cyclic lysophosphatidic acid/hydrogenated lecithin/chole sterol: 1%;
substances to be encapsulated (referred to as "minerals" in Table 1) and a chelating agent for dissolving these substances;
each cationic polymer prepared in Example 1: 3%; and
PCA ethyl cocoyl arginate as a preservative: 0.02%.

As exemplary negatively charged materials, minerals (hot-spring sinter extract) were used for the substances to be encapsulated. Stability was evaluated for cationized vesicles encapsulating minerals formed from various cationic polymers, in which 0.01% to 1% of minerals (hot-spring sinter extract) were encapsulated by using a chelating agent. Changes in zeta potential in the presence or absence of Polyquaternium-64 as a cationizing substance were measured. Aggregation and precipitation were visually observed. The results are shown in Table 1. The particle size was evaluated as large when the z-average or the main peak in scattering intensity is 200 nm or more.

Common vesicles (Extrasome CP-7M, 13 and 127 of the table) had a negative zeta potential. In known cationized vesicles (Lipidure C, 17 of the table), the stability severely deteriorated when 0.01% of minerals were encapsulated, thereby causing precipitation and aggregation. Cationized vesicles encapsulating minerals that have a zeta potential of +20 mV or more and that exhibit stability for a few days or more were obtained by using a cationic polymer with a cationic monomer proportion of 50% or more and a molecular weight of 600,000 or more.

[Table 1]

| | Labeling name · Composition | 13 | 127 | 17 | 38 | 31 | 126 | 114 | 103 | 40 |
|---|---|---|---|---|---|---|---|---|---|---|
| Extrasome CP-7M | Hydrogenated sodium cyclic lysophosphatidic acid/hydrogenated lecithin/cholesterol | 1 | 1 | 1 | 1 | 1 | 1 | 1 | 1 | 1 |
| Lipidure C | MPC/cation =70/30 Molecular weight 600,000 | - | - | 3 | - | - | - | - | - | - |
| Composition-changed Lipidure C MPC/Blemmer QA copolymer | MPC/cation = 67/33 Molecular weight 300,000 | - | - | - | - | 3 | - | - | - | - |
| | MPC/cation = 50/50 Molecular weight 700,000 | - | - | - | - | - | 3 | - | - | - |
| | MPC/cation = 45/55 Molecular weight 100,000 | - | - | - | - | - | - | 3 | - | - |
| | MPC/cation = 45/55 Molecular weight 200,000 | - | - | - | - | - | - | - | 3 | - |
| | MPC/cation = 45/55 Molecular weight 600,000 | - | - | - | - | - | - | - | - | 3 |
| | MPC/cation = 45/55 Molecular weight 700,000 | - | - | - | 3 | - | - | - | - | - |
| | MPC/cation = 45/55 Molecular weight 2,000,000 | - | - | - | - | - | - | - | - | - |
| | MPC/cation = 30/70 Molecular weight 700,000 | - | - | - | - | - | - | - | - | - |
| | MPC/cation = 18/82 Molecular weight 100,000 | - | - | - | - | - | - | - | - | - |
| | MPC/cation = 18/82 Molecular weight 400,000 | - | - | - | - | - | - | - | - | - |
| MPC/MOETAC copolymer | MPC/cation = 45/55 Molecular weight 700,000 | - | - | - | - | - | - | - | - | - |
| MPC/DMAEA-BQ copolymer | MPC/cation = 45/55 Molecular weight 700,000 | - | - | - | - | - | - | - | - | - |
| Hot-spring sinter extract | Minerals | - | 0.01 | 0.01 | - | 0.01 | 0.01 | 0.01 | 0.01 | 0.01 |
| Chelest PA-SD | Pentetic acid | - | - | - | - | - | - | - | - | - |
| CAE | PCA ethyl cocoyl arginate | 0.02 | 0.02 | 0.02 | 0.02 | 0.02 | 0.02 | 0.02 | 0.02 | 0.02 |
| Zeta potential (mV) | | -45.8 | -8.2 | 22.3 | 49.4 | 25 | 21.7 | 25.7 | 23.5 | 32.9 |
| Particle size | Z-average (nm) | 38.4 | 73.0 | 390.8 | 83.7 | 747.6 | 91.1 | 511.8 | 85.1 | 164.8 |
| | First peak in scattering intensity (nm) | 74.9 | 84.0 | 1113.0 | 114.7 | 1108.0 | 109.1 | 1152.0 | 100.3 | 194.0 |
| | | Negative zeta potential | Negative zeta potential | Large particle size, Aggregation/precipitation | | Large particle size, Gradual aggregation/precipitation | | Large particle size, Aggregation/precipitation on next day | Gradual aggregation/precipitation | |

| | Labeling name · Composition | 41 | 41SB10 | 42 | 125 | 104 | 86 | 112 | 113 | 67 |
|---|---|---|---|---|---|---|---|---|---|---|
| Extrasome CP-7M | Hydrogenated sodium cyclic lysophosphatidic acid /hydrogenated lecithin/cholesterol | 1 | 1 | 1 | 1 | 1 | 1 | 1 | 1 | 1 |
| Lipidure C | MPC/cation =70/30 Molecular weight 600,000 | - | - | - | - | - | - | - | - | - |
| Composition-changed Lipidure C  MPC/Blemmer QA copolymer | MPC/cation = 67/33 Molecular weight 300,000 | - | - | - | - | - | - | - | - | - |
| | MPC/cation = 50/50 Molecular weight 700,000 | - | - | - | - | - | - | - | - | - |
| | MPC/cation = 45/55 Molecular weight 100,000 | - | - | - | - | - | - | - | - | - |
| | MPC/cation = 45/55 Molecular weight 200,000 | - | - | - | - | - | - | - | - | - |
| | MPC/cation = 45/55 Molecular weight 600,000 | - | - | - | - | - | - | - | - | - |
| | MPC/cation = 45/55 Molecular weight 700,000 | 3 | 3 | - | - | - | - | - | - | 3 |
| | MPC/cation = 45/55 Molecular weight 2,000,000 | - | - | 3 | - | - | - | - | - | - |
| | MPC/cation = 30/70 Molecular weight 700,000 | - | - | - | 3 | - | - | - | - | - |
| | MPC/cation = 18/82 Molecular weight 100,000 | - | - | - | - | 3 | - | - | - | - |
| | MPC/cation = 18/82 Molecular weight 400,000 | - | - | - | - | - | 3 | - | - | - |
| MPC/MOETAC copolymer | MPC/cation = 45/55 Molecular weight 700,000 | - | - | - | - | - | - | 3 | - | - |
| MPC/DMAEA-BQ copolymer | MPC/cation = 45/55 Molecular weight 700,000 | - | - | - | - | - | - | - | 3 | - |
| Hot-spring sinter extract | Minerals | 0.01 | 0.01 | 0.01 | 0.01 | 0.01 | 0.01 | 0.01 | 0.01 | 1 |
| Chelest PA-SD | Pentetic acid | - | - | - | - | - | - | - | - | 1 |
| CAE | PCA ethyl cocoyl arginate | 0.02 | 0.02 | 0.02 | 0.02 | 0.02 | 0.02 | 0.02 | 0.02 | 0.02 |
| | Zeta potential (mV) | 30.2 | 26.5 | 31.0 | 32.0 | 30.6 | 34.3 | 32.8 | 23.1 | 26.2 |
| Particle size | Z-average (nm) | 140.0 | 121.9 | 187.0 | 88.5 | 126.8 | 75.2 | 141.4 | 788.9 | 60.7 |
| | First peak in scattering intensity (nm) | 159.9 | 181.7 | 250.5 | 109.7 | 173.4 | 91.3 | 172.6 | 70.7 | 70.9 |
| | | | | Slightly large particle size | | | | | Large particle size | |

[Example 3: Storage Method for Cationized Vesicles Encapsulating Minerals by Freeze Drying]

The cationized vesicles encapsulating minerals prepared in Example 2, 10% Cluster Dextrin® (highly branched cyclic dextrin), and purified water were mixed at a ratio of 1:3:6, stirred, and then freeze-dried at -20°C or lower.

The particle size and zeta potential were maintained in a stable manner in both a state before freezing, in which the original solution of the cationized vesicles encapsulating minerals was mixed with 10% Cluster Dextrin® for freeze drying, and a re-dissolved state with purified water after freeze drying (no date disclosed).

[Example 4: Efficacy Test]

A skin permeation/penetration test was performed by using a fluorescent dye as a substance to be encapsulated. Episkin™ large model (Episkin) was set in a 12-well plate containing 2 mL of PBS, added with 300 μL of 0.01% calcein and SLV, MLV, CSLV, and CMLV encapsulating 0.01% calcein from the stratum corneum side, and cultured at 37°C for 6 hours and 24 hours. Subsequently, each skin was rinsed with purified water, transferred to a tube together with stainless steel beads and 1 mL of purified water, homogenized in TissueLyser, and centrifuged. The fluorescence intensity (Ex/Em = 494/520 nm) of the resulting supernatant was measured to calculate the amount of calcein in the skin, and the skin permeation effects were evaluated. Moreover, the fluorescence intensity (Ex/Em = 494/520 nm) was similarly measured for PBS in the 12-well plate to evaluate the skin penetration effects.

The results are shown in Table 2. Compared with SLV and MLV, CSLV and CMLV had high skin permeation/skin penetration effects, respectively.

[Table 2]

**EP 3 721 866 A1**

| Sample name | | 1 | 2 | 3 | Ave | SD |
|---|---|---|---|---|---|---|
| Calcein aqueous solution | Cal | 0.019 | 0.014 | - | **0.017** | 0.004 |
| MLV encapsulating calcein | Cal-MLV | 0.096 | 0.101 | - | **0.099** | 0.004 |
| CMLV encapsulating calcein | Cal-CMLV | 0.231 | 0.167 | 0.125 | **0.174** | 0.053 |
| SLV encapsulating calcein | Cal-SLV | 0.170 | 0.136 | - | **0.153** | 0.024 |
| CSLV encapsulating calcein | Cal-CSLV | 0.570 | 0.729 | 0.633 | **0.644** | 0.080 |

[Example 5: Electron Microscope Observation]

SLV, MLV, CSLV, and CMLV were observed under a transmission electron microscope. As a result, it was confirmed that SLV and CSLV had a unilamellar structure, whereas MLV and CMLV had a multilamellar structure.

[Example 6: Patch Test]

A closed patch test was conducted for 20 Japanese subjects for 24 hours by using patch tapes coated with a cationic polymer, CSLV, and CMLV. Vaseline, physiological saline, and purified water were used for a negative control. One hour after removing the patch tapes, the state of erythema was determined as compared with the negative control and the rating criteria below, and the skin irritation index was calculated by the following formula. The samples were classified from the skin irritation index in accordance with the table below.

$$\text{Skin irritation index} = \text{sum of scores/number of subjects} \times 100$$

[Table 3]

| Rating criteria | Score | Reactions |
|---|---|---|
| - | 0 | No reaction |
| + | 0.5 | Mild erythema |
| + | 1.0 | Erythema |
| ++ | 2.0 | Erythema + edema, papule |
| +++ | 3.0 | Erythema + edema + papule + small blister |
| ++++ | 4.0 | Large blister |

The classification criteria are shown in Table 4 below.

[Table 4]

| Skin irritation index | Classification |
|---|---|
| 5.0 or less | Safe product |
| 5.0 to 15.0 | Acceptable product |
| 15.0 to 30.0 | Product to be improved |
| 30.0 or more | Risk product |

All of the three samples were classified as "safe product."

[Exemplary Production of Cationized Vesicles Encapsulating Active Ingredients (Pretreatment)]

Cationized Vesicles Encapsulating Minerals 1

[Table 5]

| | | | |
|---|---|---|---|
| A | Water | | balance |
| | Hydrogenated lecithin | | 1.00 |
| | Cholesterol | | |
| | Hydrogenated sodium cyclic lysophosphatidic acid | | |
| | Sea salt | | 0.45 |
| | Seawater | | 0.55 |
| | PCA ethyl cocoyl arginate | | 0.02 |
| | Polvquaternium-64 | | 3.00 |
| | Total (wt%) | | 100.00 |

Production Method

The aqueous phase A is heated to 75°C, emulsified with a homogenizer, cooled to 35°C, and subjected to high-pressure treatment (220 MPa × 5 passes) to yield cationized vesicles encapsulating minerals 1.

Cationized Vesicles Encapsulating Minerals 2

[Table 6]

| | | | |
|---|---|---|---|
| A | Water | | balance |
| | Hydrogenated lecithin | | 1.00 |
| | Cholesterol | | |
| | Hydrogenated sodium cyclic lysophosphatidic acid | | |
| | Mineral salt | | 1.00 |
| | PCA ethyl cocoyl arginate | | 0.02 |
| | Polvquaternium-64 | | 3.00 |
| | Pentetic acid | | 1.00 |
| | Total (wt%) | | 100.0000 |

Production Method

The aqueous phase A is heated to 75°C, emulsified with a homogenizer, cooled to 35°C, and subjected to high-pressure treatment (220 MPa × 5 passes) to yield cationized vesicles encapsulating minerals 2.

Cationized Vesicles Encapsulating Vitamin C Derivative

[Table 7]

|   | Water | balance |
|---|---|---|
| A | Hydrogenated lecithin | |
| | Cholesterol | 1.00 |
| | Hydrogenated sodium cyclic lysophosphatidic acid | |
| | Sodium ascorbyl phosphate | 5.00 |
| | PCA ethyl cocoyl arginate | 0.02 |
| | Polyquaternium-64 | 3.00 |
|   | Total (wt%) | 100.0000 |

Production Method

The aqueous phase A is heated to 75°C, emulsified with a homogenizer, cooled to 35°C, and subjected to high-pressure treatment (220 MPa × 5 passes) to yield cationized vesicles encapsulating a vitamin.

[Exemplary Production of Cosmetics]

Cream

[Table 8]

|   | Water | balance |
|---|---|---|
| A | Glycerol | 5.0000 |
| | Carbomer | 0.2000 |
| | Xanthan gum | 0.1000 |
| | Raffinose | 2.0000 |
| | 1,2-Hexanediol | 1.3500 |
| | Acrylates/C10-30 alkyl acrylate crosspolymer | 0.1500 |
| | Glucosylrutin | 0.0200 |
| | Phytic acid | 0.1000 |
| | Serine | 0.0100 |
| | Glycine | 0.0100 |
| | BG | 0.0149 |
| | *Eisenia Arborea* extract | 0.0005 |
| | Cationized vesicles encapsulating minerals 1 | 20.0000 |
| | Cyclopentasiloxane | 2.0000 |
| | Dimethicone | 3.0000 |
| | Squalane | 0.0990 |
| | Stearyl glycyrrhetinate | 0.3000 |
| | Polyglyceryl-3 polydimethylsiloxyethyl dimethicone | 0.5000 |
| | Cyclopentasiloxane | 0.2175 |
| | Dimethiconol | 0.0325 |
| | Batyl alcohol | 1.1250 |

(continued)

| B | Stearic acid | 1.1250 |
|---|---|---|
| | Caprylic/capric triglyceride | 0.1500 |
| | Lecithin | 0.1000 |
| | Behenyl alcohol | 2.5000 |
| | Shear butter | 5.0000 |
| | Octyldodecanol | 0.1000 |
| | PEG-10 dimethicone | 0.5000 |
| | *Argania spinose* kernel oil | 0.1000 |
| | Tocopherol | 0.1000 |
| C | Arginine | 0.5000 |
| | Water | 4.5000 |
| | Total (wt%) | 100.0000 |

Production Method

The aqueous phase A is heated to 75°C and dissolved with stirring. The oil phase B is heated to 85°C and dissolved with stirring. The cationized vesicles encapsulating minerals 1 and oil phase B are added to the aqueous phase A, heated to 80°C, emulsified with a homogenizer, cooled to 45°C, neutralized by adding the aqueous phase C, and cooled to 35°C to yield a cream.

Lotion

[Table 9]

| | Water | balance |
|---|---|---|
| A | Propanediol | 5.00000 |
| | 1,2- Hexanediol | 1.00000 |
| | Glycerol | 5.00000 |
| | Raffinose | 2.00000 |
| | Carbomer | 0.05000 |
| | Acrylates/C10-30 alkyl acrylate crosspolymer | 0.10000 |
| | Hydroxypropylcyclodextrin | 0.10000 |
| | PEG-20 | 2.00000 |
| | Dipotassium glycyrrhizate | 0.30000 |
| | PCA ethyl cocoyl arginate | 0.02000 |
| | Pyridoxine HCl | 0.01000 |
| | Glycine | 0.01000 |
| | Serine | 0.01000 |
| | BG | 0.00297 |
| | Simethicone | 0.01200 |
| B | Cationized vesicles encapsulating minerals 1 | 17.50000 |

(continued)

| | | |
|---|---|---|
| C | AMPD | 0.12000 |
| | Total (wt%) | 100.00000 |

Production Method

The aqueous phase A is heated to 75°C and dissolved with stirring. The aqueous phase A is then cooled to 40°C, added with the B of the Pretreatment process, stirred, neutralized by further adding the aqueous phase C, and cooled to 35°C to yield a lotion.

Essence

[Table 10]

| | | |
|---|---|---|
| | Plum fruit steam distilled water | balance |
| | Methyl gluceth-10 | 1.18 |
| | Glycerol | 0.65 |
| | 1,2-Hexanediol | 0.74 |
| | Diglycerol | 0.79 |
| | Lysine HCl | 0.20 |
| | PCA-Na | 0.50 |
| | Sugarcane extract | 0.05 |
| | Raffinose | 1.20 |
| | Serine | 0.20 |
| | BG | 5.00 |
| | Alanine | 0.20 |
| A | Arginine | 0.20 |
| | Aspartic acid | 0.05 |
| | Aminocaproic acid | 0.30 |
| | Biotin | 0.00 |
| | Pyridoxine HCl | 0.50 |
| | Acetylhydroxyproline | 0.15 |
| | Acetylglucosamine | 0.15 |
| | Glycine | 0.15 |
| | Aminobutyric acid | 0.15 |
| | Dipotassium glycyrrhizate | 0.50 |
| | PEG-240/HDI copolymer bis-decyltetradeceth-20 ether | 0.60 |
| | Panthenol | 0.20 |
| | Fermented plum juice | 3.00 |
| B | Glutathione | 0.07 |
| | Hydrolyzed eggshell membrane | 0.06 |
| C | Cationized vesicles encapsulating vitamin C derivative | 20.00 |

(continued)

| | Total (wt%) | 100.00 |
|---|---|---|

Production Method

The aqueous phase A is heated to 80°C, dissolved with stirring, cooled to 40°C, added with the aqueous phase B then with the C of the Pretreatment process for essence, and cooled to 35°C to yield an essence.

Mineral Cream

[Table 11]

| A | Water | balance |
|---|---|---|
| | Raffinose | 3.000 |
| | Sodium stearoxy PG-hydroxyethylcellulose sulfonate | 0.050 |
| | PEG-240/HDI copolymer bis-decyltetradeceth-20 ether | 0.050 |
| | Glycerol | 5.000 |
| | Carbomer | 0.150 |
| | 1,2-Hexanediol | 1.000 |
| B | Cationized vesicles encapsulating minerals 1 | 15.000 |
| C | Cationized vesicles encapsulating minerals 2 | 15.000 |
| E | Phytosteryl/octyldodecyl lauroyl glutamate | 3.500 |
| | Dimethicone | 2.500 |
| | Behenyl alcohol | 3.000 |
| | Batyl alcohol | 1.575 |
| | Stearic acid | 1.575 |
| | Caprylic/capric triglyceride | 0.210 |
| | Lecithin | 0.140 |
| | Stearyl glycyrrhetinate | 0.300 |
| | PEG-10 dimethicone | 1.000 |
| | Polyglyceryl-3 polydimethylsiloxyethyl dimethicone | 0.500 |
| | Tocopherol | 0.100 |
| | PEG-150 stearate | 0.250 |
| | Disteardimonium hectorite | 0.150 |
| F | AMPD | 0.150 |
| | Total (wt%) | 100.0000 |

Production Method

The aqueous phase A is heated to 80°C, dissolved with stirring, added with the B and C of the Pretreatment process, heated to 80°C, and dissolved with stirring. The oil phase E is heated to 90°C and dissolved with stirring. The oil phase E is then added to the aqueous phase A, heated to 80°C, emulsified with a homogenizer, cooled to 45°C, neutralized by adding the aqueous phase F, and cooled to 35°C to yield a cream.

Mineral Lotion

[Table 12]

| A | Water | balance |
|---|---|---|
| | BG | 2.8000 |
| | 1,2-Hexanediol | 1.7000 |
| | Sodium acrylate/acryloyldimethyltaurate/dimethylacrylamide crosspolymer | 0.0625 |
| | Polyacrylate crosspolymer-6 | 0.2625 |
| | Panthenol | 0.1000 |
| | Simethicone | 0.0120 |
| | Hydroxypropyltrimonium hyaluronate | 0.0050 |
| | Raffinose | 3.0000 |
| | Acetylhydroxyproline | 0.0400 |
| | Disodium adenosine triphosphate | 0.0010 |
| | Biotin | 0.0010 |
| | Dipotassium glycyrrhizate | 0.2000 |
| | *Tremella fuciformis* polysaccharide | 0.0200 |
| | Acetylglucosamine | 0.0400 |
| B | Cationized vesicles encapsulating minerals 1 | 15.0000 |
| C | Cationized vesicles encapsulating minerals 2 | 15.0000 |
| D | AMPD | 0.0400 |
| | Total (wt%) | 100.0000 |

Production Method

The aqueous phase A is heated to 80°C, dissolved with stirring, cooled to 40°C, added with the B and C of the Pretreatment process, stirred, neutralized by adding the aqueous phase D, and cooled to 35°C to yield a mineral lotion.

Shampoo

[Table 13]

| | Water | |
|---|---|---|
| A | Polyquaternium-47 | 0.400 |
| | Polyquaternium-10 | 0.850 |
| | Cocamide MEA | 1.350 |

(continued)

| | | | |
|---|---|---|---|
| | B | Sodium ascorbyl phosphate | 1.000 |
| | | Sodium citrate | 0.030 |
| | | Citric acid | 0.300 |
| | | BG | 1.000 |
| | | 1,2-Hexanediol | 1.000 |
| | | Polyquaternium-64 | 0.300 |
| | | Methylisothiazolinone | 0.010 |
| | | Glycosyl trehalose | 0.050 |
| | | Hydrogenated starch hydrolysate | 0.030 |
| | | Cationized vesicles encapsulating minerals 2 | 10.000 |
| | C | Sodium methyl cocoyl taurate | 3.500 |
| | | Sodium lauroyl methylaminopropionate | 4.750 |
| | | EDTA-2Na | 0.005 |
| | | Sodium cocoyl apple amino acids | 1.000 |
| | | Cocamidopropyl betaine | 1.000 |
| | | Lauramidopropyl hydroxysultaine | 1.000 |
| | | NaCl | 0.500 |
| | D | Perfume | 0.300 |
| | | Total (wt%) | 100.000 |

Production Method

The aqueous phase A is heated to 80°C and dissolved with stirring. The aqueous phase B is heated to 75°C, dissolved with stirring, and added to the aqueous phase A. The aqueous phase C is heated to 60°C, dissolved with stirring, added to the aqueous phase A, cooled to 40°C, added with the aqueous phase D, and cooled to 35°C to yield a shampoo.

Body Milk

[Table 14]

| | Water | balance |
|---|---|---|
| A | Glycerol | 10.000 |
| | BG | 4.000 |
| | Sorbitol | 1.500 |
| | Methylparaben | 0.200 |
| | Xanthan gum | 0.150 |
| | Carbomer | 0.150 |
| | Polyquaternium-64 | 0.300 |
| | 1,2-Hexanediol | 0.500 |
| | Raffinose | 0.500 |
| | Sodium ascorbyl phosphate | 1.000 |
| | Cationized vesicles encapsulating minerals 2 | 20.000 |
| B | Neopentyl glycol dicaprate | 14.000 |
| | Jojoba oil | 2.000 |
| | Stearyl glycyrrhetinate | 0.300 |
| | Tocopherol | 0.100 |
| | Phytosteryl macadamiate | 0.100 |
| | *Mortierella* oil | 0.200 |
| | Cetyl-PG hydroxyethyl palmitamide | 0.180 |
| | Behenyl alcohol | 0.400 |
| | Batyl alcohol | 0.180 |
| | Stearic acid | 0.180 |
| | Lecithin | 16.000 |
| | Caprylic/capric triglyceride | 24.000 |
| | Ceteth-6 | 0.900 |
| | Propylparaben | 0.200 |
| | Dimethicone | 4.000 |
| | Diphenylsiloxy phenyl trimethicone | 2.000 |
| C | Arginine | 0.500 |
| D | Superoxide dismutase | 0.001 |
| | Phytic acid | 0.300 |
| | Total (wt%) | 100.000 |

Production Method

The aqueous phase A is heated to 70°C and dissolved with stirring. The oil phase B is heated to 80°C and dissolved with stirring. The cationized vesicles encapsulating minerals 2 and oil phase B is added to the aqueous phase A, heated to 75°C, emulsified with a homogenizer, neutralized by adding the aqueous phase C, cooled to 40°C, added with the aqueous phase D, and cooled to 35°C to yield a body milk.

**Claims**

1. Cationized vesicles that are cationized with a cationic polymer containing 2-methacryloyloxyethyl phosphorylcholine and a cationic monomer and that are used for cosmetics through addition thereto, wherein a proportion of the cationic monomer in the cationic polymer is 50% or more.

2. The cationized vesicles according to Claim 1, wherein the vesicles are unilamellar vesicles or multilamellar vesicles.

3. The cationized vesicles according to Claim 1 or 2, wherein the cationic monomer is (2-hydroxy-3-methacryloyloxypropyl)trimethylammonium chloride, [2-(methacryloyloxy)ethyl]trimethylammonium chloride, or N,N-dimethylamino ethylacrylate, benzyl chloride quarternary.

4. Cationized vesicles encapsulating an active ingredient, wherein the active ingredient is for cosmetics and is held by the cationized vesicles according to any one of Claims 1 to 3.

5. The cationized vesicles encapsulating an active ingredient according to Claim 4, wherein the active ingredient is a mineral.

6. The cationized vesicles encapsulating an active ingredient according to Claim 4, wherein the active ingredient is vitamin C or a vitamin C derivative.

7. The cationized vesicles according to any one of Claims 1 to 3 or the cationized vesicles encapsulating an active ingredient according to any one of Claims 4 to 6, wherein a zeta potential is +20 mV or more.

8. The cationized vesicles according to any one of Claims 1 to 3 or the cationized vesicles encapsulating an active ingredient according to any one of Claims 4 to 6, wherein the vesicles are freeze-dried.

9. A cosmetic comprising cationized vesicles, wherein the cationized vesicles are the cationized vesicles according to any one of Claims 1 to 3, the cationized vesicles encapsulating an active ingredient according to any one of Claims 4 to 6, or the freeze-dried cationized vesicles or cationized vesicles encapsulating an active ingredient according to Claim 8.

10. The cosmetic according to Claim 9, wherein the cosmetic has adsorption properties to the skin and hair and is applied topically.

11. A method for a beauty treatment comprising applying the cosmetic according to Claim 9 to the skin or hair.

# INTERNATIONAL SEARCH REPORT

International application No.

PCT/JP2017/044249

## A. CLASSIFICATION OF SUBJECT MATTER

Int. Cl. A61K8/81(2006.01)i, A61K8/02(2006.01)i, A61Q5/00(2006.01)i, A61Q19/00(2006.01)i

According to International Patent Classification (IPC) or to both national classification and IPC

## B. FIELDS SEARCHED

Minimum documentation searched (classification system followed by classification symbols)

Int. Cl. A61K8/81, A61K8/02, A61Q5/00, A61Q19/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

CAplus/MEDLINE/EMBASE/BIOSIS (STN), JSTPlus/JMEDPlus/JST7580 (JDreamIII), Mintel GNPD

## C. DOCUMENTS CONSIDERED TO BE RELEVANT

| Category* | Citation of document, with indication, where appropriate, of the relevant passages | Relevant to claim No. |
|---|---|---|
| X | JP 2016-108285 A (WAMILES COSMETICS KK) 20 June | 1-11 |
| Y | 2016, examples 1-4, claims, paragraphs [0009], [0018], [0022], [0044], [0045] (Family: none) | 1-11 |
| Y | JP 2015-189762 A (KOBAYASHI PHARMACEUTICAL CO., LTD.) 02 November 2015, pharmaceutical preparation examples 11-20, 28-34, claims, paragraphs [0010], [0038], [0040], [0041], [0045], [0064], [0069], [0070] (Family: none) | 1-11 |
| Y | JP 6-178930 A (POLA CHEMICAL INDUSTRIES INC.) 28 June 1994, examples, (in particular, example 4), paragraphs [0019], [0046] (Family: none) | 1-11 |

☒ Further documents are listed in the continuation of Box C.  ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 19.02.2018 | 27.02.2018 |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2017/044249 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2005-336127 A (DOCTOR PROGRAM KK) 08 December 2005 (Family: none) | 1-11 |
| A | JP 2012-184182 A (NOF CORP.) 27 September 2012 (Family: none) | 1-11 |
| A | WO 2010/067617 A1 (NATIONAL UNIVERSITY CORPORATION HOKKAIDO UNIVERSITY) 17 June 2010 (Family: none) | 1-11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 5623076 B
- JP 2012206948 A
- JP 2007176820 A
- JP 2007176821 A
- JP 4950419 B
- JP 5220546 B
- JP 2006035036 A
- JP 2016108285 A

- JP 2007217356 A
- JP 2000290159 A
- JP 3967357 B
- JP 4795475 B
- JP 2526362 B
- JP 2012116761 A
- JP 3544505 B
- JP 2009029788 A

### Non-patent literature cited in the description

- **BANGHAM et al.** *J. Mol. Biol.,* 1965, vol. 13, 238
- *Biochem. Biophys. Res. Commun.,* 1980, vol. 94, 1367
- *J. Cell. Biol.,* 1975, vol. 66, 621
- *FEBS Lett.,* 1979, vol. 99, 210
- *N. Y. Acad. Sci.,* 1978, vol. 308, 250

- *Biochim. Biophys. Acta,* 1976, vol. 455, 322
- *Biochim. Biophys. Acta,* 1978, vol. 394, 483
- *Arch. Biochem. Biophys.,* 1981, vol. 212, 186
- *Proc. N. A. S. USA,* 1978, vol. 75, 4194
- *Third US-Japan Symposium on Drug Delivery System,* 1995